# EUROPEAN PATENT SPECIFICATION

(11) **EP 3 917 405 B1**
(45) Date of publication and mention of the grant of the patent: **18.09.2024**
(21) Application number: 19797519.6
(22) Date of filing: 15.10.2019
(51) Int. Cl.: A61B 8/00

(54) **INSTRUMENTED ULTRASOUND PROBES FOR MACHINE-LEARNING GENERATED REAL-TIME SONOGRAPHER FEEDBACK**
INSTRUMENTIERTE ULTRASCHALLSONDEN FÜR DURCH MASCHINENLERNEN ERZEUGTES ECHTZEITFEEDBACK EINES SONOGRAFEN
SONDES ULTRASONORES INSTRUMENTÉES POUR RETOUR D'INFORMATIONS DE SONOGRAPHE EN TEMPS RÉEL GÉNÉRÉE PAR APPRENTISSAGE AUTOMATIQUE

(30) Priority: 04.02.2019 US 201962800825 P
(43) Date of publication of application: 08.12.2021
(73) Proprietor: Google LLC, Mountain View, CA 94043 (US)
(72) Inventor: STARNS, Alex, Mountain View, CA 94043 (US); TSE, Daniel, Mountain View, CA 94043 (US); SHETTY, Shravya, Mountain View, CA 94043 (US)
(74) Representative: Marks & Clerk GST
(86) International application number: PCT/US2019/056248
(87) International publication number: WO 2020/162989

(56) References cited:
- WO-A1-2018/226377
- US-A1- 2017 262 982
- US-A1- 2018 153 505
- US-A1- 2018 225 993

## Description

### Background

This disclosure relates to an ultrasound probe with position, orientation and optionally other inertial and environmental sensors, a machine learning model and a feedback mechanism to help an ultrasound technologist improve image quality of ultrasound images.

Ultrasound requires a highly skilled and experienced technologist to derive high quality images. An experienced technologist will intuitively manage many different parameters in producing a high-quality image, including position of the patient's body, position of the organ or tissue in question, position of the ultrasound probe, orientation of the ultrasound probe, type of ultrasound probe, pressure applied and speed of sweep.

However, in some geographic regions, including the developing world, highly skilled ultrasound technologists are either rare or nonexistent, and often ultrasound images are obtained by a person of moderate or even minimal skill. Hence, such images often have limited diagnostic usefulness. There is a need in the art for an automated system for assisting a technologist in capturing high quality images. This disclosure remedies this situation.

Document WO 2018/226377 A1 describes systems and methods for identifying and navigating anatomical objects using a visualization guide.

### Summary

An ultrasound system is described which includes an ultrasound probe that is provisioned with position sensors, such as an off-the-shelf inertial sensor unit having a combination of accelerometers and/or gyroscopes, and position sensors. The position sensors provide position and orientation information of the ultrasound probe in 3D space during use. The system uses machine learning models which are trained to correlate ultrasound images (such as image content or image quality) with ultrasound probe position and orientation. The system further provides a feedback generator receiving position and orientation data from the sensors and a display for providing real-time feedback to the technologist with suggestions for adjusting the probe position, orientation, pressure, sweep speed, and other parameters to improve the image quality. The feedback display consists of directional indicia or a user interface incorporated into the probe, and can further include lighted buttons or arrows paced on the ultrasound probe, a display on the ultrasound probe, displays on a monitor showing the ultrasound image, displays on an external computing device such as a personal computer (e.g., laptop computer) connected to the position and orientation sensors over an air interface, verbal instructions projected from a speaker, or combination thereof.

The feedback enables the technologist to quickly improve their skills, to produce better quality images and to 'up-level' from amateur to professional technologist more quickly. It may also allow unskilled or novice medical staff to produce high-quality sonograms, thereby improving access to this essential diagnostic tool and potentially reducing the cost of this access.

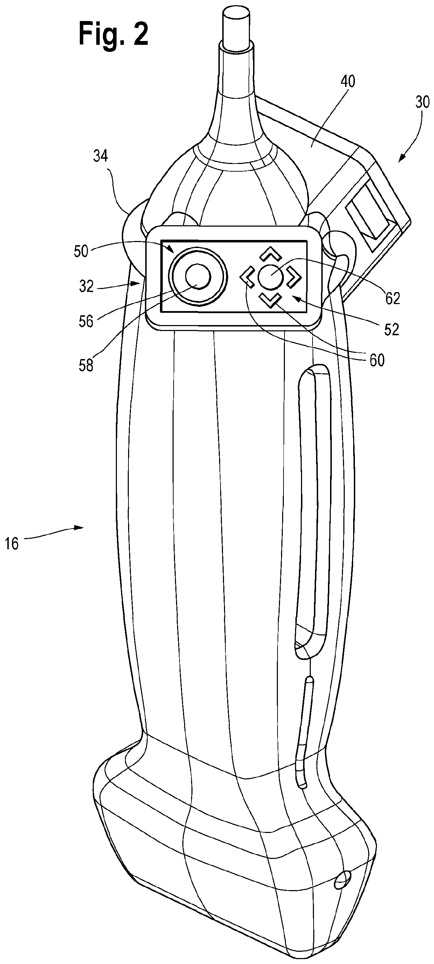

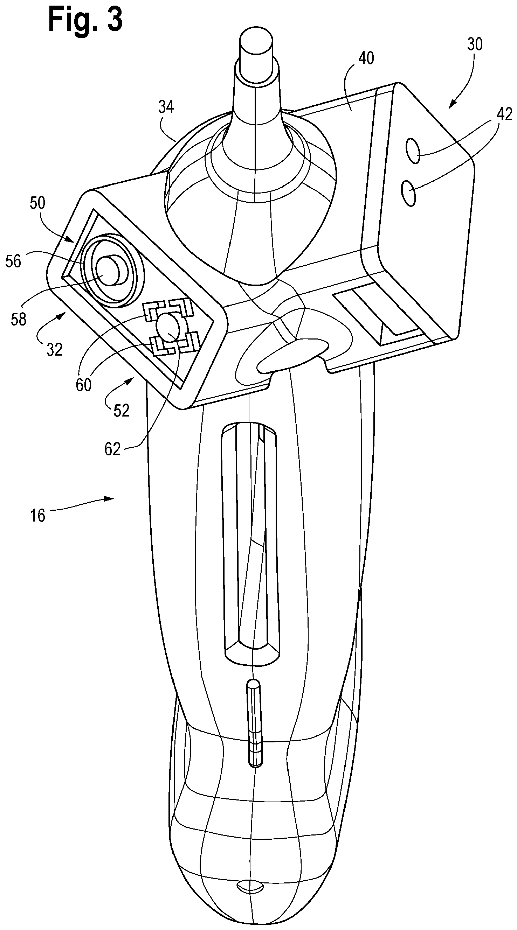

In one possible configuration, it is envisioned that the ultrasound probes' orientation can be inferred relatively precisely from the content and quality of the image using the trained machine learning model. In this situation, no hardware modifications to the ultrasound systems in use is necessary in order to generate the feedback; in particular the use of the position sensors in the embodiment of Figures 2 and 3 is not required and such position sensors would only be used in generating the training set for model generation. The machine learning model is able to infer position and orientation from the ultrasound images alone and then, based on the inferred current position and orientation, a feedback generator provides suggested feedback to the operator, for example as a difference in X,Y, and Z position and orientation vector V from the current position to position and orientation where high quality images were obtained in the training set. Accordingly, in this configuration, an ultrasound system is described which includes an ultrasound probe generating ultrasound image data; one or more machine learning models trained to correlate ultrasound images with probe position and orientation, wherein the one or more machine learning models receive images generated from the ultrasound probe; a feedback generator generating feedback data based on the current probe position determined by the one or more machine learning models; and a feedback display receiving the feedback data providing real-time suggestions to the user of the ultrasound probe for adjusting the probe position, orientation, pressure and/or other parameters of the ultrasound probe to improve the quality of the images generated from the ultrasound probe.

In another aspect, a method for improving ultrasound images is disclosed.

### Brief Description of the Drawings

Figure 1 is an illustration of the system of this disclosure in use.
Figure 2 is a perspective view of an ultrasound probe featuring both position and orientation sensors and a feedback display for generating feedback to the technologist to assist them in positioning the probe so as to generate high quality ultrasound images.
Figure 3 is another perspective view of the ultrasound probe of figure 2.
Figure 4 is an illustration of a feedback display that is incorporated into a display of the ultrasound image currently being captured by the technologist. As the technologist captures the ultrasound images the feedback display changes so as to continually provide updated feedback to the technologist to assist them in capturing a steady stream of high quality images.
Figure 5 is an illustration of a feedback display that is incorporated into a display of a personal (e.g., laptop) computer which is proximate to the technologist during the ultrasound session, e.g., placed on the ultrasound cart. The laptop may also incorporate a speaker which provides spoken (audio) feedback, such as "move a little to the left", "sweep slower", "move the probe closer to the head", etc.
Figure 6A is a block diagram of an ultrasound system in which the ultrasound probe is provided with a feedback display, as is shown in Figures 2 and 3.
Figure 6B is a block diagram of an ultrasound system in which an external laptop computer is provided with a feedback display, as is shown in Figure 5.
Figure 6C is a block diagram of an ultrasound system in which the ultrasound image display is provided with a feedback display, as is shown in Figure 4.
Figure 7 is an illustration of a training set for training a machine learning model of this disclosure.
Figure 8 is a diagram showing a process for training the model with the training set of Figure 7 to infer ultrasound probe position from a set of annotated ultrasound images with position and orientation data.
Figure 9 is a block diagram showing the trained machine learning model and a feedback generator which generates feedback instructions for display in accordance with one of the options of Figures 6A-6C from current image data and current probe position.
Figure 10 is a flow chart showing the operation of the method for improving ultrasound images.

### Detailed Description

We describe in detail below ultrasound probes with position and orientation sensors and machine-learning generated real-time sonographer feedback with the goal of providing a system that assists the ultrasound technologist in obtaining high quality ultrasound images. We further disclose in this document a machine learning training set for model development by instrumenting an ultrasound probe with position and orientation sensors, so sonogram images captured can be associated accurately with the position and orientation of the probe at the time of capture. In this way, machine learning models may be able to associate image content and quality to the type, position and orientation of the probe with respect to the patient. These models would then be able infer probe position and orientation based on the content and quality of an image, and produce a position/orientation correction for the person conducting the scan. When presented with this position correction suggestion, the person conducting the scan could adjust the probe position and orientation and get a better quality image resulting in more efficient, lower cost, and higher quality diagnostic outcomes for the patient. In our envisioned tool, the ultrasound probe would have, either built in from manufacture, or added after-market, an accelerometer and gyroscope sensor package, an onboard computer to process data and run the machine learning model, and a feedback device such as lights, auditory indicators or a digital display to provide feedback to the person conducting the scan. There any may ways to implement this approach. Here are several:
a) Manufacture the sensors and computer into the housing of the probe, with data integration included such that processing of the image and fusion with the sensor data occurs within the ultrasound device itself. Feedback to the technologist could occur via the screen already included with the ultrasound device.
b) Modify an existing ultrasound probe to embed the sensors and computers into the housing and intercept the ultrasound image data at time of capture to directly analyze and provide feedback to the technologist via an onboard screen or separate display device.
c) Connect an external computer to the ultrasound machine, and connect it wirelessly (e.g. by Bluetooth) to the instrumented ultrasound probe. This external computer would associate the ultrasound images and probe orientation data and provide feedback to an onboard display visible to the technologist. As used in this document, the term "instrumented probe" or "instrumented ultrasound probe" means an ultrasound probe which is augmented with inertial position and orientation sensors as described in this document.

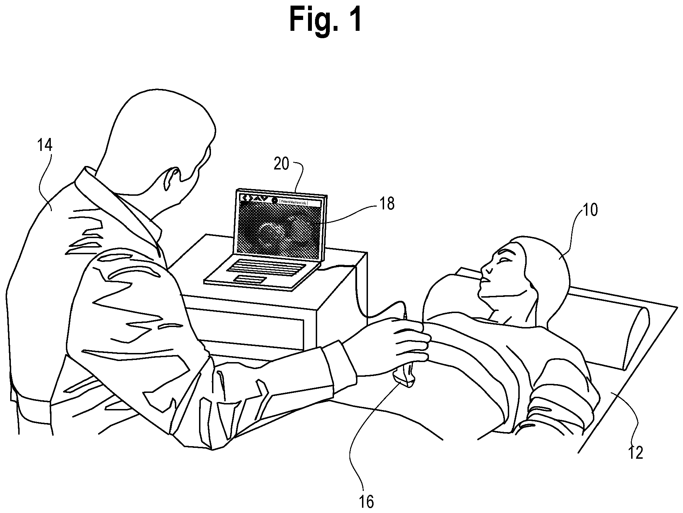

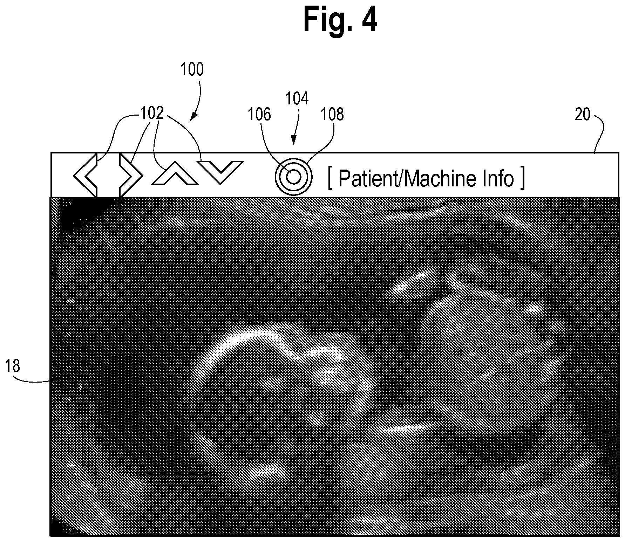

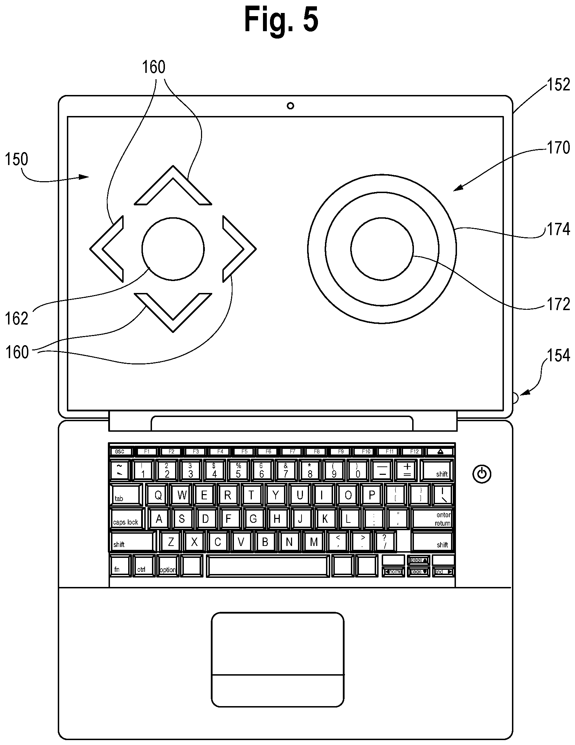

Figure 1 is an illustration of one example the system of this disclosure in use. A patient 10 is positioned on a table 12 and a technologist 14 using an ultrasound probe 16 is generating images 18 which are displayed on a monitor 20 connected to the probe 16. The probe 16 is shown in more detail in Figures 2 and 3, in the embodiment where position and orientation sensors are included in the probe 16, and in one configuration such sensors are not required. The monitor 20 displays both the ultrasound images and the feedback suggestions. A machine learning model and feedback generator is incorporated into the computer processing of the ultrasound system/monitor. The feedback generator provides feedback suggestions for how the technologist 14 can improve the quality of the images by reorienting or repositioning the probe against the patient's body which are then displayed to the operator. Examples of the feedback displays are shown in Figures 4 and 5 and will be described in some detail below. As another example, the feedback display can be built into the ultrasound probe 16 as will be discussed in Figures 2 and 3, or displayed on a separate personal computer such as a laptop.

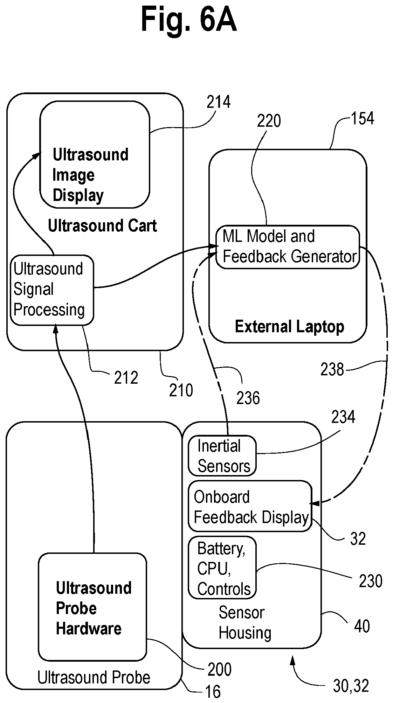

Figures 2 and 3 are perspective views of the ultrasound probe 16 of Figure 1. The probe 16 is otherwise conventional except for the addition of two modules 30 and 32 which are connected to a mounting ring 34 which slips over or clamps onto the probe housing 36. The module 30 includes a housing 40 which contains a battery (power supply) for inertial position and orientation sensors (not shown in Figures 2 and 3, but shown in Figures 6A-6C) which are contained within the housing. The module also includes a central processing unit (CPU) and other processors for conducting certain computing operations as will be explained in conjunction with the block diagrams of Figures 6A-6C later in this document. The housing also includes one or more operation buttons 42 for functions such as power on/off, reset, calibration, etc.

The module 32 provides for a position and orientation feedback display 50 and 52. The module 32 is configured to face the operator when the probe is gripped by the technologist's hand and used in normal operation. The display 50 is configured to be analogous to a "bubble level" and is designed to provide orientation feedback and includes an outer ring 56 and a central circle 58. The outer ring is fixed in position and the central circle 'floats' with an angular and radial parameter. The angular parameter indicates in what direction the operator should tilt the probe, and the radial parameter indicates how much tilt should be applied. For example, an angular parameter of 0 degrees ("up") would indicate to the operator to tilt the probe away from themselves and 180 degrees ("down") would indicate to the operator that they should tilt it towards themselves. The radial parameter would be reactive - as the operator approaches the correct tilt, the central circle would re-center itself, indicating the operator had achieved the desired probe orientation.

The display 52 includes a set of directional indicia, in the form of four chevrons 60 pointing in different directions, and a central circle 62. One or more of the chevrons 60 light up to indicate suggested position adjustments (along the surface of the patient's body, e.g., move up (closer to the head) down, right or left). The central circle 62 changes color to indicate more or less pressure is needed to improve the quality of the ultrasound images.

While the embodiment of the ring 34 and modules 30, 32 shown in Figures 2 and 3 is specifically adapted for retrofit use onto an existing ultrasound probe, it will be appreciated that the feedback module 32 and the electronics module 30 could have different form factors and further they could be incorporated into the housing for the probe 16 at the time of manufacture and may be built into the probe housing itself. The form factor for the sensors and feedback displays is not considered critical and can vary widely from the details of this disclosure.

Figure 4 is an illustration of a feedback display 100 that is incorporated into a display of the ultrasound image currently being captured by the technologist and displayed on a monitor 20. As the technologist captures the ultrasound images the feedback display 100 changes so as to continually provide updated feedback to the technologist to assist them in capturing a steady stream of high quality images. In this embodiment, the display 100 includes left, right, up and down chevrons 102 which are selectively highlighted to indicate the operator should move the ultrasound probe to the left or right or up (towards the head) or down to improve image quality. At right, a 'bubble level' display 104 indicates orientation feedback. The central circle 106 'drifts' towards the outer ring 108, indicating in which direction the probe should be tilted. By trying to keep the circle 106 centered in the ring, the operator can maintain the correct probe orientation. A further field 110 to the right is for display of patient/machine information and the details are not important. While Figure 4 shows one possible implementation of a feedback display implemented on the built-in monitor of the ultrasound machine itself (or secondary computer attached to the ultrasound machine), the position and orientation feedback indicators 100 and 104 could be displayed along the top margin, as shown in Figure 4, or another suitable location (e.g. the lower right corner of the monitor).

Figure 5 is an illustration of a feedback display 150 that is incorporated into a display 152 of a laptop computer 154 which is proximate to the technologist during the ultrasound session, e.g., placed on the ultrasound cart. The laptop 154 may also incorporate a speaker which provides spoken (audio) feedback, such as "move a little to the left", "sweep slower", "move the probe closer to the head", "press deeper" etc. The feedback display 150 includes a set of chevrons 160 and central circle 162. As the same for the other embodiments, one or more of the chevrons 160 light up to indicate suggested position adjustments (along the surface of the patient's body). The central circle 162 changes color to indicate more or less pressure. The 'bubble level' display 170 indicates orientation feedback. The central circle 172 'drifts' towards the outer ring 174, indicating in which direction the probe should be tilted to improve image quality. By attempting to keep the circle centered in the ring, the operator can maintain the correct probe orientation.

Figures 6A-6C are block diagrams of an ultrasound system in accordance with three different possible embodiments. In Figure 6A, the ultrasound probe is provided with a feedback display, as is shown in Figures 2 and 3. In this embodiment, the ultrasound probe 16 includes ultrasound probe hardware 200 (conventional) which provide ultrasound image data to an ultrasound cart 210 which includes ultrasound signal processing circuitry 212 providing images for display on an ultrasound image display 214 (conventional). The images generated in the signal processing circuitry 212 are provided to an external computer (e.g., laptop computer) 154 which includes a machine learning (ML) model and a feedback generator, together shown as 220.

The ultrasound probe 16 also has an inertial sensor module 30 and an onboard feedback display 32 as per Figures 2 and 3. The inertial sensors 234 include a wireless transmitter (e.g., Bluetooth) which provide position and orientation data over an air interface 236 to the ML model and feedback generator 220 in the laptop 154. Data representing the feedback generated in the module 220 (such as position or orientation adjustment suggestions) are provided over an air interface 238 to the onboard feedback display 32 for generation of visual feedback suggestions to the operator on the display 32 of the ultrasound probe in Figures 2 and 3. For example, the feedback suggestions could be activating one of the chevrons 60 in Figure 3 to move the ultrasound probe to the right or left. The sensor housing 40 also includes a battery for the inertial and position sensors 234, a central processing unit and additional control hardware and software shown at 230, such as on/off switches, a calibration switch, etc. the details of which are not important.

The inertial position and orientation sensors 234 in one configuration consist of a miniaturized inertial measurement sensors (e.g., a combination of accelerometers and/or gyroscopes, currently embodied in MEMS technology) and a wireless transmitter (e.g., WIFI or Bluetooth) that functions to relay information as to the current position of the ultrasound probe to an external computing device, e.g., the laptop computer 154 of Figure 6A.

The inertial measurement sensor 234 and wireless transmitter can be integrated as a single unit, e.g., the MetaMotionC Sensor from MBient Labs, which is based on a Bosch BMI160 chipset. High volume applications, including smart phones and gaming controllers, have driven down cost and size of accelerometers and gyros. The applications have also driven increased integration with wireless components and decreased power consumption. Further considerations for an implementation are minimization of software integration effort, fool-proof, and long battery life. In the illustrated embodiment, we used a MetaMotionC inertial measurement sensor with built-in wireless transmitter. Mbient Labs, the manufacturer, has developed a platform with several compact, wireless motion sensors and a Linux-compatible software development kit. The underlying Bosch chipset in the MetaMotionC provides advanced functionality and computing resources such as sensor fusion that converts raw signals into an absolute orientation vector. The resolution, range, and accuracy of the inertial measurement unit in the sensor are more than sufficient for detecting ultrasound probe orientation and position.

### Software integration

Mbient Labs provides a hub (Raspberry Pi based) for initial software development. This may also come in handy for other applications such as component testing. Free and open source software development kits (SDK's) are available in a variety of languages, including C++, Java, and Python. Many examples are provided. Apps, such as MetaBase, are also available for iOS and Android. This allows rapid set-up of the sensor. Data can be streamed to the external computing device (e.g., smartphone) or logged on device and downloaded later.

### Sensor operation

The MetaMotionC board is built around the Nordic RF52 system-on-chip platform, which integrates wireless communication (Bluetooth), CPU and sensor communication/logging. Circuit diagrams showing the internal components are publicly available. All inertial measurement sensors needed for the present uses are provided by a Bosch BMI160 chip in the unit. This device includes 3-axis accelerometers and gyroscopes (both based on MEMS technology). It also includes a 3-axis magnetometer and computational features to synthesize signals from multiple sensors and report absolute orientation.

### Wireless

Bluetooth (BLE) on the Nordic chip provides a wireless link to access sensor data. Range: Line of sight indoors is ~10m. Battery life: The MetaMotionC is powered by a Li-ion coin-cell battery (CR2032, typically -200mAh). Power management features are built into the primary power consuming chips (BMI160 and nRF5832). These features will likely need to be managed to achieve >1-year battery life. For example, there is a lower power accelerometer command in the iOS API.

### Configuration

The device can be configured in 3 ways. Note that each MetaMotionC is configured as a slave peripheral, which can only be connected to 1 master device at a time. Beacon: Sensor data is advertised to the world to be picked up by any Client (e.g. Smartphone or BLE dongle). Stream: Sensor data is sent live to the Client while connected. Log: Sensor data is kept in the MetaMotionC memory (8 MB) to be downloaded at a later time.

### Determining orientation

The Bosch chip determines absolute sensor orientation, as indicated above. Gyroscopic drift is one of the key considerations for sensor accuracy, as all 3 axes of the gyro are sensitive to rotational acceleration and not absolute angle. Ultrasound heading (or clocking angle) can be derived from the z-axis of the accelerometer (where gravity provides asymmetry) and the magnetometer in the device. If the sensor were positioned in a horizontal plane, it's z-axis would be parallel to the direction of the earth's gravitational force. This degenerate condition eliminates sensitivity of the accelerometer to pure rotation about the z-axis. Fortunately, ultrasound probes are commonly tilted by ~15 degrees from horizontal. This introduces a component of gravity to the x and y axes, which is orientation-dependent.

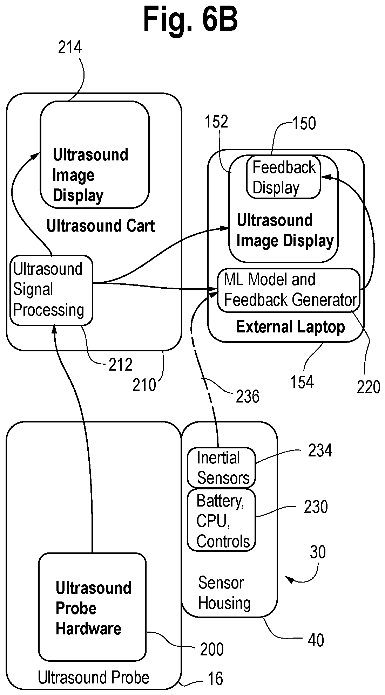

Figure 6B is a block diagram of an ultrasound system in which an external laptop computer is provided with a feedback display, as is shown in Figure 5. in this configuration, the ultrasound probe includes the ultrasound probe hardware 200 and has the same configuration as in Figure 6A. The probe includes an inertial sensor module 30, the housing of which 40 contains the inertial sensors 234 and the battery, CPU and controls module 230 as explained in Figure 6A. The inertial sensors 234 send position and orientation data over the air interface 236 (using Bluetooth, WIFI, or the equivalent) to an external laptop 154. The ultrasound cart 212 includes ultrasound signal processing circuitry 212 generating ultrasound images for display on the ultrasound image display 214. The ultrasound images are fed to both the ML model and feedback generator 220 and to an ultrasound image display 152 (See Figure 5). Feedback generated by the feedback generator 220 is presented on the display as indicated at 150 in Figure 5.

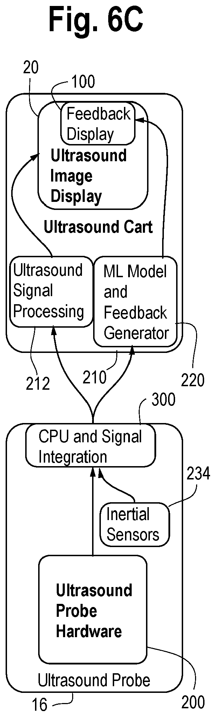

Figure 6C is a block diagram of an ultrasound system in which the ultrasound image display is provided with a feedback display, as is shown in Figure 4. The inertial sensors 234 are incorporated into the ultrasound probe. The position and orientation data from the sensors are supplied ot the CPU 300 performing signal integration with the ultrasound image data from the probe hardware 200. Ultrasound image data is sent to both the ultrasound image signal processing circuitry 212 and to the ML model and feedback generator 220. The ultrasound signal processing circuitry generates ultrasound images which are presented on the display 20. Feedback suggestions generated by the feedback generator 220 are presented in the feedback display region 100 of the display 20, as indicated in Figure 4. Note: in this embodiment there is no need for an external computer running the machine learning models and the feedback generator, as the central processing unit(s) of the ultrasound machine on the cart 210 are configured with these models and generator as indicated at 220. Note further that that the inertial sensors 234 do not include wireless transmit or receive functions, and instead the inertial sensors are hard wired to the CPU 300 for signal integration. This embodiment is an example of an ultrasound probe itself which is configured at the time of manufacture with the inertial sensors and there is no need for a retrofit sensor configuration as shown in Figures 2 and 3.

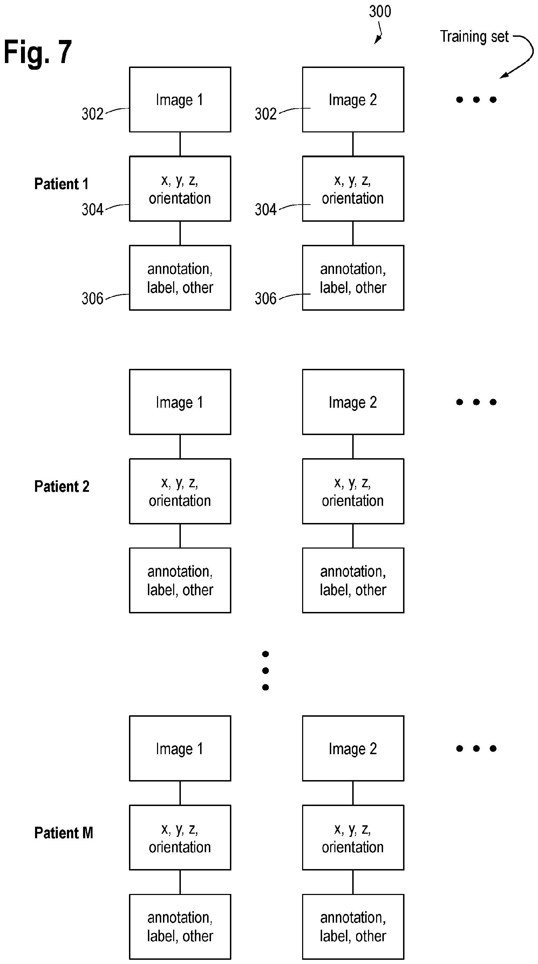

While the above description is focused on the end user situation of assisting an operator in obtaining high quality ultrasound images, there is a preliminary step of generating a machine learning training set and ML model development. This step is achieved by instrumenting an ultrasound probe with position and orientation sensors (such as using the design of Figures 2 and 3), so that sonogram images are captured which can be associated accurately with the position and orientation of the ultrasound probe at the time of image capture. It is envisioned that a very large number (e.g., > 100,000, and possibly a million or more) of ground truth annotated ultrasound images will be obtained from skilled operators on a variety human subjects, on a variety of tissue or organ types, together with probe 3D position and orientation information for each image obtained from precisely calibrated and accurate position and orientation sensors. Such a set of images is referred to as the training set, and one example of such a set is shown in Figure 7 at 300. In particular, for a given patient, such as patient 1, there are a sequence of images 1, 2 ....N (N >100), 302, and each image 302 is associated with both X,Y,Z positional information as well as orientation (vector V) of the probe at the time of capture of the image, 304. Additionally, each image is associated with metadata or annotations 306, such as for example the age and sex of the patient, the tissue being subject to ultrasound, and optionally a ground truth label that is human-assigned to the image, such as an identification of specific tissue or structures shown in the label, an image quality metric, and so on. Prior to capturing the images, there may be an initial set-up or calibration step in which patient age, sex, tissue type or region, and other data are entered into a computer that is used to store the ultrasound images. The ultrasound probe position and orientation measurements may also be subject to initial calibration, such as by defining an origin in three dimensional space and setting a vector V to a null position when in a vertical orientation at the origin. Once this initial calibration and set up steps are performed, the subject (patient 1) is subject to ultrasound scanning and a stream of images are acquired, each with the positional information from the inertial sensors in the probe. The operator may review the images and add ground truth labels, either during the ultrasound scanning or afterwards.

The process of acquiring ultrasound images and data as explained above is repeated for many different patients, hundreds or even thousands, and for each patient a set of images with positional information and annotations/metadata/labels is acquired as shown in Figure 7. Several different training sets as per Figure 7 could also be obtained in the situation where there are different machine learning models trained to associate images (and specifically image quality) with probe position and orientation due to anatomical differences in the type of tissue or region of the body being subject to sonography, for example one machine learning model for ultrasound of pregnant women to image the fetus, and another machine learning model for ultrasound of upper thoracic structures such as the heart and lungs.

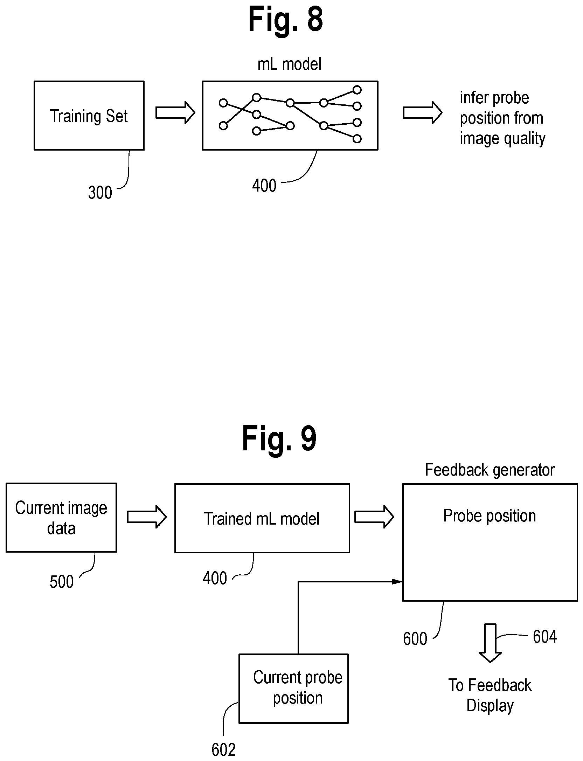

At shown in Figure 8 the resulting training set 300 is provided as input to a machine learning model 400 which may take the form of a neural network, e.g., deep convolutional neural network. The model 400 is trained such that sonogram images can be associated accurately with the position and orientation of the probe at the time of capture. In this way, machine learning models may be able to associate image content and quality to the type, position and orientation of the probe with respect to the patient.

The manner of use of the model 400 trained in Figure 8 is shown in Figure 9. The model 400 is able to infer probe position and orientation based on the content and quality of an image 500. The inferred probe position is supplied to a feedback generator 600, which produces a position/orientation correction for the person conducting the scan taking into account the current probe position 602 measured by the inertial sensors (Figures 2, 3, 6A-6C) in the probe in order to guide the person to obtaining higher quality images. The feedback data 604 generated by the feedback generator 600 is supplied to the feedback display of the system, whether positioned on the probe itself (Figures 2, 3), on the display monitor of the ultrasound system (Figure 4) or on a separate computer (154, Figure 5).

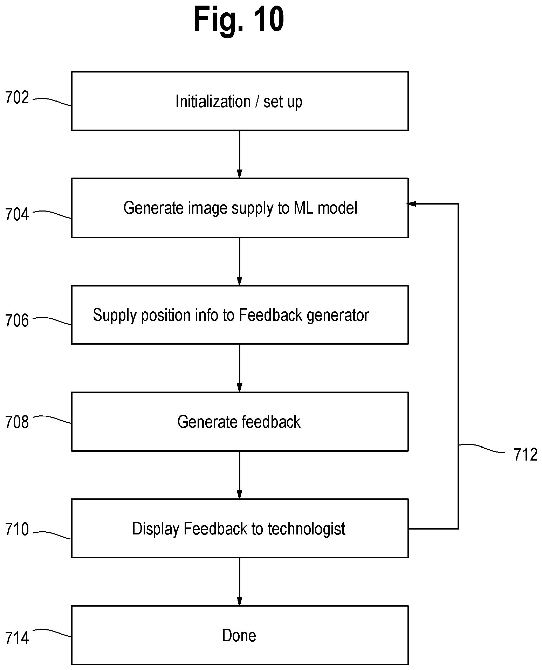

Figure 10 is a flow chart 700 showing the operation of the method for improving ultrasound images. At step 702, there is an initial calibration of the position and orientation sensors and set-up (e.g., performed on a computer associated with the ultrasound system) in which the patient data is entered and the type of tissue or region of the body being subject to sonography is identified. This step is needed so that that correct machine learning model is identified and used.

At step 704, the ultrasound probe generates ultrasound image data and the images are supplied to the machine learning model 400 of Figure 9. At step 706, the position and orientation information obtained by the sensors is supplied to the feedback generator 600 of Figure 600. At step 708, the feedback generator generates feedback data, for example position and orientation corrections based on the differences between the current probe position and orientation and the position and orientation of the probe when high quality images were obtained in the training set. At step 710 the feedback data is displayed on a feedback display in accordance with Figures 2, 3, 4 or 5 to thereby aid the technologist in obtaining better quality images. The process loops back as indicated at 712 and steps 704, 706, 708 and 710 repeat during the ultrasound session. When the session is over, the process ends as indicated at 714.

In one embodiment, the ultrasound probe would have, either built in from manufacture, or added after-market, an accelerometer and gyroscope sensor package (as explained above), an onboard computer to process data and run the machine learning model and feedback generator, and a feedback device such as lights, auditory indicators or a digital display to provide feedback to the person conducting the scan. In this embodiment there is no need for the external personal computer, as the machine learning models and feedback generation is done by processors included in the probe, e.g., in accordance with the configuration of Figures 2 and 3.

### Further considerations

It is envisioned that the ultrasound probes' orientation can be inferred relatively precisely from the content and quality of the image using the trained machine learning model of Figure 9. If this is the case, no hardware modifications to the ultrasound systems in use is necessary in order to generate the feedback; in particular the use of the position sensors in the embodiment of Figures 2 and 3 is not required and such position sensors would only be used in generating the training set of Figure 7. The ML model 400 (Figure 8) is able to infer position and orientation from the image and then, based on the inferred current position and orientation, provide suggested feedback to the operator, for example as a difference in X, Y, and Z position and orientation vector V from the current position to position and orientation where high quality images were obtained in the training set.

In another embodiment, for example with the configuration of Figures 2 and 3 and the block diagrams of Figures 6A-7C, and Figure 9, a probe with instrumentation (inertial position and orientation sensors) could still be used to provide this input to the feedback generator which then couples with an ML model fusing that data with the imagery. In this case, after-market hardware systems could be added to ultrasound probes for a modest cost (e.g., using lower quality sensors such as the ones specifically mentioned in this document). In this particular situation, that the ML model 400 (Figure 9) infers the probe is at position X₁, Y₁, Z₁, orientation vector V₁, and based on its training more optimal images would be captured if the probe was moved to position X₂, Y₂, Z₂, orientation vector V₂, and therefore the feedback command is generated by the feedback generator 600 as the difference between these positions/orientations. The feedback generator then takes into account the actual current position and orientation generated by the position sensors to compute the Δ X, Y, Z position and Δ V orientation movement to place the ultrasound probe in the more optimal position.

It is further noted that the instrumented systems used to create the initial training set of Figure 7, however, may well require much more expensive and finely tuned sensors and may not be low cost, off the shelf items.

In summary, in one configuration image content alone is enough to infer position and orientation and generate feedback to provide operator guidance. In another possible configuration, training sets derived from high-end position/orientation sensors enable ML-guided ultrasound with probes augmented with minimally expensive position/orientation sensors, as shown in Figures 2, 3, 6A-6C and 9. In another configuration, only expensive lab-grade instrumented systems allow the system to provide guidance to the operator. While this embodiment does not lend itself as much to widespread, low cost adoption of the present methods could still be useful for teaching and training applications.

The term "position sensors" is intended to refer to one or more sensors which determine the position and orientation information of an ultrasound probe in 3D space during use and may include for example off-the-shelf position and orientation sensors such as an inertial sensor unit using having a combination of accelerometers and/or gyroscopes and position sensors. One example of a "position sensors" is a miniaturized inertial measurement sensor incorporating MEMS (Micro Electro-Mechanical Systems) technology, such as the MBient Laboratories chip specifically mentioned previously. Other types of position sensors are commercially available to persons skilled in the art and therefore the specification is not intended to be limited to a certain type, construction, design or manufacturer of position sensors. In one possible embodiment the position sensors are integrated with a wireless transmitter (as in the case of the MBient Laboratories chip) so as to enable transmission of the position and orientation data over the air to an external computing device, e.g., a laptop computer or smart phone, which then runs the machine learning model and determines the feedback to assist the operator make adjustments on the position, orientation, pressure, etc. as explained above to improve the image quality.

In one possible configuration, the machine learning model and feedback generator could be implemented in a remote server system with calls to and from the server system via an application programming interface (API). For example, the ultrasound probe could include a processing unit to send image data via the API to the machine learning model and receive feedback data from the feedback generate via the API. Alternatively, a personal computer such as the laptop shown in the figures could include an API interface to the remote server system in which case the feedback generator and machine learning model are remotely located but the feedback is displayed on the display of the personal computer.

## Claims

1. An ultrasound system, comprising in combination:
an ultrasound probe (16) generating ultrasound image data;
one or more machine learning models trained to correlate ultrasound images with probe position and orientation, wherein the one or more machine learning models receive images generated from the ultrasound probe;
a feedback generator generating feedback data based on a current probe position determined by the one or more machine learning models; and
a feedback display receiving the feedback data providing real-time suggestions to the user of the ultrasound probe for adjusting the probe position, orientation, or pressure of the ultrasound probe to improve the quality of the images generated from the ultrasound probe,
**characterized in that** the feedback display comprises directional indicia incorporated into or on the ultrasound probe.

2. The system of claim 1, wherein the ultrasound probe is provisioned with one or more position sensors generating real time position and orientation data as to the position of the ultrasound probe and orientation in three-dimensional space during use of the probe.

3. The system of claim 1, wherein the directional indicia comprise lighted buttons or arrows placed on the ultrasound probe.

4. The system of claim 1, wherein the feedback display comprises a display and prompts shown on the display and wherein the display is incorporated into or on the ultrasound probe.

5. The system of claim 1, wherein the feedback display further comprises a display on a monitor and prompts shown on the display, and wherein the display further presents displays of images generated by the ultrasound probe.

6. The system of claim 1, wherein the feedback display further comprises a speaker generating audible prompts for the user.

7. The system of any of claim 2, wherein the one or more position sensors comprises a miniaturized inertial measurement sensor incorporating MEMS (Micro Electro-Mechanical Systems) technology.

8. The system of claim 7, wherein the miniaturized inertial measurement sensor is further configured with a wireless transmitter transmitting position and orientation data to an external computing device.

9. The system of claim 8, wherein the external computing device comprises a monitor displaying the ultrasound images captured by the ultrasound probe.

10. A method for improving ultrasound images, comprising the steps of:
generating positional information as to the position and orientation of an ultrasound probe (16) while the probe is generating the ultrasound images;
supplying the positional information and the images to a machine learning model which is trained to correlate ultrasound images with ultrasound probe position and orientation and
generating feedback based on a current probe position determined by the machine learning model in the form of suggestions for changing the position or orientation of the ultrasound probe in order to improve the quality of the images, **characterized in that**
the generating comprises generating suggestions for display on a user interface incorporated into the probe.

11. The method of claim 10, wherein the generating step further comprises the step of generating audible prompts with a speaker.

12. The method of claim 10, wherein the generating step comprises the step of activating one or more directional indicia incorporated into or on the ultrasound probe.

13. The method of claim10, wherein the generating step further comprises the step of generating a display on an ultrasound monitor providing guidance to the operator of the ultrasound probe to move the probe or change the orientation of the probe in three dimensional space in substantial real time while the images are generated.

14. The method of claim 10, wherein the generating step further comprises the step of generating a display on a personal computer in wireless communication with the position sensors providing guidance to the operator of the ultrasound probe to move the probe or change the orientation of the probe in three dimensional space in substantial real time while the images are generated.

## Patentansprüche

1. Ultraschallsystem, umfassend in Kombination:
eine Ultraschallsonde (16), die Ultraschallbilddaten erzeugt;
ein oder mehrere Maschinenlernmodelle, die trainiert wurden, um Ultraschallbilder mit der Sondenposition und -ausrichtung zu korrelieren, wobei das eine oder die mehreren Maschinenlernmodelle die von der Ultraschallsonde erzeugten Bilder empfangen;
einen Feedback-Generator, der Feedback-Daten basierend auf einer aktuellen Sondenposition erzeugt, die von dem einen oder mehreren Maschinenlernmodellen bestimmt wird; und
eine Feedback-Anzeige, die die Feedback-Daten empfängt, die dem Benutzer der Ultraschallsonde Echtzeit-Vorschläge zum Einstellen der/des Sondenposition, -ausrichtung oder -drucks der Ultraschallsonde bereitstellt, um die Qualität der von der Ultraschallsonde erzeugten Bilder zu verbessern, **dadurch gekennzeichnet, dass** die Feedback-Anzeige Richtungsangaben umfasst, die in oder an der Ultraschallsonde aufgenommen sind.

2. System nach Anspruch 1, wobei die Ultraschallsonde mit einem oder mehreren Positionssensoren ausgestattet ist, die Positions- und Orientierungsdaten in Echtzeit erzeugen, die die Position der Ultraschallsonde und die Orientierung im dreidimensionalen Raum während der Verwendung der Sonde betreffen.

3. System nach Anspruch 1, wobei die Richtungsangaben beleuchtete Knöpfe oder Pfeile umfassen, die an der Ultraschallsonde platziert sind.

4. System nach Anspruch 1, wobei die Feedback-Anzeige eine Anzeige und auf der Anzeige dargestellte Aufforderungen umfasst und wobei die Anzeige in oder auf der Ultraschallsonde aufgenommen wird.

5. System nach Anspruch 1, wobei die Feedback-Anzeige ferner eine Anzeige auf einem Bildschirm und auf der Anzeige dargestellte Aufforderungen umfasst und wobei die Anzeige ferner Anzeigen von Bildern anzeigt, die durch die Ultraschallsonde erzeugt werden.

6. System nach Anspruch 1, wobei die Feedback-Anzeige ferner einen Lautsprecher umfasst, der hörbare Aufforderungen für den Benutzer erzeugt.

7. System nach einem der Ansprüche 2, wobei der eine oder die mehreren Positionssensoren einen miniaturisierten Trägheitsmesssensor umfassen, der die MEMS-Technologie (Micro Electro-Mechanical Systems) aufnimmt.

8. System nach Anspruch 7, wobei der miniaturisierte Trägheitsmesssensor ferner mit einem drahtlosen Übertrager konfiguriert ist, der Positions- und Orientierungsdaten an eine externe Rechenvorrichtung überträgt.

9. System nach Anspruch 8, wobei die externe Rechenvorrichtung einen Bildschirm umfasst, der die von der Ultraschallsonde erfassten Ultraschallbilder anzeigt.

10. Verfahren zum Verbessern von Ultraschallbildern, umfassend Folgende Schritte:
Erzeugen von Informationen über die Position und Ausrichtung einer Ultraschallsonde (16), während die Sonde die Ultraschallbilder erzeugt;
Liefern der Informationen über die Position und der Bilder an ein Maschinenlernmodell, das trainiert ist, Ultraschallbilder mit der Position und Ausrichtung der Ultraschallsonde zu korrelieren, und
Erzeugen von Feedback basierend auf einer durch das Maschinenlernmodell bestimmten aktuellen Sondenposition in Form von Vorschlägen zum Ändern der Position oder Ausrichtung der Ultraschallsonde, um die Qualität der Bilder zu verbessern,
**dadurch gekennzeichnet, dass** das Erzeugen das Erzeugen von Vorschlägen zur Anzeige auf einer in die Sonde aufgenommenen Benutzerschnittstelle umfasst.

11. Verfahren nach Anspruch 10, wobei der Erzeugungsschritt ferner den Schritt des Erzeugens hörbarer Aufforderungen mit einem Lautsprecher umfasst.

12. Verfahren nach Anspruch 10, wobei der Erzeugungsschritt den Schritt zum Aktivieren einer oder mehrerer Richtungsangaben umfasst, die in oder an der Ultraschallsonde aufgenommen sind.

13. Verfahren nach Anspruch 10, wobei das Erzeugen ferner den Schritt des Erzeugens einer Anzeige auf einer Ultraschallsonde umfasst, die dem Bediener der Ultraschallsonde eine Anleitung bereitstellt, um die Sonde zu bewegen oder die Ausrichtung der Sonde im dreidimensionalen Raum im Wesentlichen in Echtzeit zu ändern, während die Bilder erzeugt werden.

14. Verfahren nach Anspruch 10, wobei der Schritt des Erzeugens ferner den Schritt des Erzeugens einer Anzeige auf einem Personalcomputer umfasst, der in drahtloser Kommunikation mit den Positionssensoren steht und dem Bediener der Ultraschallsonde eine Anleitung bereitstellt, um die Sonde zu bewegen oder die Ausrichtung der Sonde im dreidimensionalen Raum im Wesentlichen in Echtzeit zu ändern, während die Bilder erzeugt werden.

## Revendications

1. Système ultrasonore, comprenant en combinaison :
une sonde ultrasonore (16) générant des données d'image ultrasonore ;
un ou plusieurs modèles d'apprentissage automatique formés pour corréler des images ultrasonores avec une position et une orientation de sonde, dans lequel l'un ou les plusieurs modèles d'apprentissage automatique reçoivent des images générées par la sonde ultrasonore ;
un générateur de rétroaction générant des données de rétroaction sur la base d'une position de sonde actuelle déterminée par l'un ou les plusieurs modèles d'apprentissage automatique ; et
un affichage de rétroaction recevant les données de rétroaction fournissant des suggestions en temps réel à l'utilisateur de la sonde ultrasonore pour ajuster la position, l'orientation de sonde ou la pression de la sonde ultrasonore pour améliorer la qualité des images générées à partir de la sonde ultrasonore,
**caractérisé en ce que** l'affichage de rétroaction comprend des indices directionnels incorporés dans ou sur la sonde ultrasonore.

2. Système selon la revendication 1, dans lequel la sonde ultrasonore est équipée d'un ou de plusieurs capteurs de position générant des données de position et d'orientation en temps réel quant à la position de la sonde ultrasonore et à l'orientation dans l'espace tridimensionnel pendant l'utilisation de la sonde.

3. Système selon la revendication 1, dans lequel les indices directionnels comprennent des boutons ou des flèches lumineux placés sur la sonde ultrasonore.

4. Système selon la revendication 1, dans lequel l'affichage de rétroaction comprend un affichage et des invites affichées sur l'affichage et dans lequel l'affichage est incorporé dans ou sur la sonde ultrasonore.

5. Système selon la revendication 1, dans lequel l'affichage de rétroaction comprend également un affichage sur un moniteur et des invites affichées sur l'affichage, et dans lequel l'affichage présente également des affichages d'images générées par la sonde ultrasonore.

6. Système selon la revendication 1, dans lequel l'affichage de rétroaction comprend également un haut-parleur générant des invites sonores pour l'utilisateur.

7. Système selon l'une quelconque de la revendication 2, dans lequel l'un ou les plusieurs capteurs de position comprennent un capteur de mesure inertielle miniaturisé incorporant la technologie MEMS (Micro Electro-Mechanical Systems).

8. Système selon la revendication 7, dans lequel le capteur de mesure inertielle miniaturisé est également configuré avec un émetteur sans fil transmettant des données de position et d'orientation à un dispositif informatique externe.

9. Système selon la revendication 8, dans lequel le dispositif informatique externe comprend un moniteur affichant les images ultrasonores capturées par la sonde ultrasonore.

10. Procédé d'amélioration d'images ultrasonores, comprenant les étapes de :
génération d'informations de position concernant la position et
l'orientation d'une sonde ultrasonore (16) pendant que la sonde génère les images ultrasonores ;
fourniture des informations de position et des images à un modèle d'apprentissage automatique qui est formé pour corréler les images ultrasonores avec la position et l'orientation de la sonde ultrasonore et
génération d'une rétroaction sur la base d'une position de sonde actuelle déterminée par le modèle d'apprentissage automatique sous la forme de suggestions de modification de la position ou
de l'orientation de la sonde ultrasonore afin d'améliorer la qualité des images, **caractérisé en ce que** la génération comprend la génération de suggestions à afficher sur une interface utilisateur intégrée à la sonde.

11. Procédé selon la revendication 10, dans lequel l'étape de génération comprend également l'étape de génération d'invites audibles avec un haut-parleur.

12. Procédé selon la revendication 10, dans lequel l'étape de génération comprend l'étape d'activation d'un ou de plusieurs indices directionnels incorporés dans ou sur la sonde ultrasonore.

13. Procédé selon la revendication 10, dans lequel l'étape de génération comprend également l'étape de génération d'un affichage sur un moniteur ultrasonore fournissant un guidage à l'opérateur de la sonde ultrasonore pour déplacer la sonde ou changer l'orientation de la sonde dans un espace tridimensionnel en temps réel substantiel pendant que les images sont générées.

14. Procédé selon la revendication 10, dans lequel l'étape de génération comprend également l'étape de génération d'un affichage sur un ordinateur personnel en communication sans fil avec les capteurs de position fournissant un guidage à l'opérateur de la sonde ultrasonore pour déplacer la sonde ou changer l'orientation de la sonde dans un espace tridimensionnel en temps réel substantiel pendant que les images sont générées.
